# EUROPEAN PATENT APPLICATION

(11) **EP 2 048 151 A1**
(43) Date of publication of application: **15.04.2009**
(21) Application number: 07019825.4
(22) Date of filing: 10.10.2007
(51) Int. Cl.: C07H 19/00, C07H 19/06, C07H 19/12, C07H 19/16

(54) **Method for producing nucleosides by direct glycosylation of the nucleoside base**

(71) Applicant: Cilag AG, 8205 Schaffhausen (CH)
(72) Inventor: Kraut, Norbert., 78250 Tengen (DE); Jungmann, Oliver., 78166 Donaueschingen (DE)
(74) Representative: Braun, André jr.

(57) **Abstract**

Summary

Method of producing a free nucleoside compound by reacting a a glycoside donor preferably a 1-halogen derivative, or 1-0-acyl, 1-o-alkyl, or an imidate preferably a trichloromethyl derivative, or a thio-alkyl derivative of a blocked monosaccharide or oligosaccharide preferably ribose and 2-desoxyribose derivatives with a protected nucleoside base, in a suitable anhydrous solvent and in the presence of a catalyst, and removing the protecting groups from said blocked nucleoside compound, wherein said catalyst is selected from the group comprising salts of an aliphatic sulphonic acid and/or salts of a optionally substituted aromatic sulphonic acid.

## Description

The present invention refers to a method of producing a nucleoside by reacting a protected, preferably silylated, nucleoside base with a glycoside donor preferably a 1-halogen derivative, or 1-O-acyl, 1-O-alkyl, or an imidate preferably a trichloromethyl derivative, or a thio-alkyl derivative of a blocked monosaccharide or oligosaccharide, preferably ribose and 2-desoxyribose derivatives, in the presence of a selected catalyst.

Nucleosides are known pharmaceutically active compounds and have been described in numerous publications. From US 4,209,613 it is known to synthesize nucleosides by silylating the corresponding nucleoside base and reacting the silylated base with a 1-0-acyl, 1-0-alkyl or 1-halogen derivative of a blocked monosaccharideor oligosaccharide in the presence of a selected catalyst by silylating the base and reacting the sugar derivative in a single step in the presence of trimethylhalosilane and/or hexamethyldisilazane. The catalysts used are e.g. selected from SnCl₄, TiCl₄, ZnCl₂, BF₃-etherate, AlCl₃ and SbCl₅, or a trialkylester of a perfluoroalkanesulfonic acid. The major disadvantage is that the catalysts mentioned in the patent (tin tetrachloride, titanium tetrachloride, zinc chloride or boron trifluoride etherate) are prone to hydrolysis giving irritant hydrolysis products like HCl or are forming insoluble oxides (TiO₂, SnO₂), which are difficult to remove from the reaction product and difficult to handle, especially on large scale production.

It has now been found that a glycoside donor preferably a 1-halogen derivative, or 1-O-acyl, 1-O-alkyl, or an imidate preferably a trichloromethyl derivative, or a thio-alkyl derivative of a blocked monosaccharide or oligosaccharide, preferably ribose and 2-desoxyribose derivatives, can be reacted with a silylated or alkylated corresponding nucleoside base in the presence of a selected catalyst, said catalyst being selected from the group comprising a salt of an aliphatic sulphonic acid and/or a salt of a optionally substituted aromatic sulphonic acid, to give excellent product yields. This type of catalyst is stable under aqueous conditions, easy to handle, does not produce irritant hydrolysis products, and can be easily removed. The selectivity of the reaction for obtaining the desired anomer, i.e. ratio of the alpha/beta anomers, and the final reaction yields are excellent as yields higher than 95%, and regularly within the range of 97-98% can be obtained.

The present invention is defined in the claims. The present invention refers to a method of producing a free nucleoside compound by reacting a glycoside donor preferably a 1-halogen derivative, or 1-O-acyl, 1-0-alkyl, or an imidate preferably a trichloromethyl derivative, or a thio-alkyl derivative preferably a thiomethyl derivative of a blocked monosaccharide or oligosaccharide preferably ribose and 2-desoxyribose derivatives, said monosaccharide or oligosaccharide being blocked by removable protecting groups, with a protected nucleoside base, in a suitable anhydrous solvent and in the presence of a catalyst, whereby a blocked nucleoside compound is obtained, and removing the protecting groups from said blocked nucleoside compound in order to obtain the free nucleoside compound, characterized in that said catalyst is selected from the group comprising salts of an aliphatic sulphonic acid and/or salts of a optionally substituted aromatic sulphonic acid.

The present invention refers also to the production of the blocked nucleoside compound as obtained from the reaction of the glycoside donor preferably a 1-halogen derivative, or 1-0-acyl, 1-0-alkyl, or an imidate preferably a trichloromethyl derivative, or a thio-alkyl derivative, preferably a thiomethyl derivative, of a blocked monosaccharide or oligosaccharide, preferably ribose and 2-desoxyribose derivatives, with the protected nucleoside base.

The catalyst used in said reaction preferably is a salt of an aliphatic sulphonic acid, preferably of methylsulphonic acid (mesylate) or of ethylsulphonic acid, or of a fluorinated aliphatic sulfonic acid, such as a salt of trifluoromethane-sulfonic acid, pentafluoroethyl-sulfonic acid, or heptafluoropropyl-sulfonic acid. Preferred of these salts are the salts of methylsulphonic acid (mesylate) and the salts of trifluoromethanesulfonic acid. Preferred are the alkali salts, and earth alkali salts. Preferred further are the lithium salts, preferably lithium methylsulphonic acid (lithium mesylate) or lithium-trifluoromethanesulfonate (LiOTf, lithiumtriflate). Generally also other salts, for example the salts of scandium, such as Sc(OTf)₃ or of copper such as Cu(OTf)₂ or of magnesium such as Mg(OTf)₂ can be used. However, the lithium salt and especially LiOTf is preferred. Further salts of known, optionally substituted, aromatic sulphonic acids may also be used; preferably the alkali salts and earth alkali salts thereof. Preferred are the alkali salts and earth alkali salts of benzenesulphonic acid (besylate), toluenesulphonic acid (tosylate), and known related acids. Preferred are the lithium salts of said aromatic sulphonic acids.

Preferred solvents to carry out the reaction according to the present invention are chlorinated solvents, such as dichloromethane, dichloroethane, chloroform, chlorobenzene, or toluene, xylol, acetonitril and/or propylene carbonate or related solvents. Preferred are chlorinated solvents. Preferred is the use of lithium-trifluoromethanesulfonate (LiOTf) in a chlorinated solvent, preferably in dichloromethane, dichloroethane, chloroform and/or chlorobenzene.

The synthesis of a glycoside donor preferably a 1-halogen derivative, or 1-O-acyl, 1-0-alkyl, or an imidate preferably a trichloromethyl derivative, or a thio-alkyl derivative of a blocked monosaccharide or oligosaccharide preferably ribose and 2-desoxyribose derivatives wherein the hydroxyl groups are being blocked by protecting groups, i.e. removable substituents, is known per se. Numerous substituents are known and can be used within the scope of the present invention. Said protecting groups preferably are selected from (C₁-C₈)alkylcarbonyl, or optionally substituted phenylcarbonyl, or optionally substituted benzylcarbonyl. Said protecting groups preferably are selected from (C₁-C₄)alkylcarbonyl, or optionally substituted phenylcarbonyl, like phenylcarbonyl, tolylcarbonyl, xylylcarbonyl or benzylcarbonyl; and is preferably acetyl or p-chloro-phenylcarbonyl.

The substituents 1-0-acyl, 1-O-alkyl, 1-halogen, 1-imidate or 1-thio-alkyl attached to the blocked monosaccharide or oligosaccharide are preferably substituents of the formulae -O-acyl(C₁-C₈), -O-alkyl(C₁-C₈) or halogen or trichloromethyl, or thiomethyl; preferably are -O-acyl(C₁-C₄), -O-alkyl(C₁-C₄) or chlorine, bromine, fluorine; preferably -O-(O)CCH₃ or chlorine, bromine, fluorine, preferably chlorine or fluorine, preferably chlorine.

The blocked monosaccharide or oligosaccharide are preferably derived from ribose, deoxyribose, arabinose, and glucose, preferably from ribose and 2-desoxyribose. Preferably all free hydroxyl groups are blocked with known protecting groups, preferably with the blocking groups as mentioned herein above, selected from (C₁-C₈)alkylcarbonyl, or optionally substituted phenylcarbonyl, or optionally substituted benzylcarbonyl. These blocking groups are known to the expert in the art.

The protected nucleoside base is protected by a removable protecting group known per se, and is preferably protected by a trimethylsilyl (TMS)-group. The preparation of protected nucleoside base compounds is known. The compound is preferably prepared by reaction of the free nucleoside base with trimethylchlorosilane or with hexamethyldisilazane. This is known to the expert in the art. Numerous nucleoside organic bases are known. Generally all these nucleoside organic bases can be reacted with the corresponding chemical compounds, e.g. trimethylchlorosilane or with hexamethyldisilazane, to yield the protected nucleoside base which can be used according to the process defined in the present invention.

Preferred nucleoside bases are halogen-derivatives, preferably chlor- or fluor-substituted derivatives, preferably fluorine substituted, and heterocyclic compounds containing five or six atoms, said heterocyclic ring containing one, two or three nitrogen atoms. Preferably the nucleoside bases are or are derived from the group comprising the following heterocyclic compounds, wherein these compounds optionally may be substituted: uracil, cytosine preferably 5-azacytosine, 6-azauracil, 2-thio-6-azauracil, thymine, N-acyl-adenine, guanine, lumazine, imidazole, pyrazine, thiazole and triazole.

In the reaction of the nucleoside base with the glycoside donor, the sugar residue is preferably linked to the nitrogen atom to form a beta-glycoside.

When reacting the protected nucleoside base with the above mentioned derivative of a blocked monosaccharide or oligosaccharide together, the reaction temperature generally is within the range of 0°C to about 90°C, preferably at about room temperature, whereby the components are reacted in about equimolar amounts and preferably with a slight excess of the protected nucleoside base. The catalyst is used preferably in a concentration of about 10-100 mol-%, calculated to the total molar presence of the two reacting components. For the expert in the art it is no problem to optimize the molar ratios of the components.

For removing the substituents from the blocked nucleoside compound in order to obtain the free nucleoside compound, containing free hydroxyl groups, known methods are used. The substituents may be preferably removed, for example, by treatment in an alcoholic solution of ammonia or alcoholates, or with aqueous or alcoholic alkali; but other known methods may be applied.

The following example illustrates the invention.

### Example 1

(A) A mixture of 5-azacytosine (20 g, 178.4 mmol), ammonium sulfate (2.4 g, 18.16 mmol), and hexamethyldisilazane (160 g, 991.3 mmol) is heated to reflux until a clear solution is obtained. The excess of hexamethyldisilazane is removed in the vacuum at 60°C.
(B) A mixture of 264 g of dichloromethane, lithium trifluoromethane sulfonate (13.92 g, 89.2 mmol) and the "chloro sugar" C-137 (3,5-Dip-chloro-benzoyl-2deoxy-alpha-ribofuranosylchloride) [46.0 g, 107.1 mmol) are prepared and added to the residue obtained in step (A).
(C) The mixture is stirred for 4 hours at ambient temperature (20-25°C). Then the solvent is removed at 40°C in the vacuum and the obtained residue is dissolved in 60 g ethyl acetate. The solution is added drop wise to a mixture of 220 g of aqueous sodium hydrogen carbonate (2.5% w-solution), 174 g ethyl acetate, 36 g cyclohexane and 70 g acetonitrile at 0°C and the obtained reaction mixture stirred for 4 h at 0°C. The precipitate of the blocked (protected) aminotriazine is filtered off, washed with aqueous sodium hydrogen carbonate (2.5% w-solution), water, and finally with a mixture of acetonitrile and ethyl acetate (1:1). Yield: 38.2 g; 49.5% [assay (beta-isomer): 70.1%; purity (beta-isomer): 64.7%].
(D) The compound corresponding to formula (III) as obtained in Section (C) is further treated with an alcoholic solution of ammonia in a known manner so that 2'-deoxy-5-azacytidine (Decitabine) is obtained in practically quantitative yield.

### Example 2

A mixture of cytosine (2.0 g, 18.0 mmol), ammonium sulfate (0.24 g, 1.82 mmol), and hexamethyldisilazane (16.13 g, 100 mmol) was heated to reflux for 45 minutes until a clear solution was obtained. Some gas evolved (ammonia). The reaction mixture was cooled to 52°C and concentrated in the vacuum whereby a colourless solid precipitated. 26.4 g of dichloromethane, lithium trifluoromethane sulfonate (2.53 g, 16.2 mmol) and the "chloro sugar" C-137 (6.96 g, 16.2 mmol) were added. The slightly beige mixture was stirred for 2 hours at ambient temperature (20-25°C), products (anomeric mixture [(β-isomer) : (α-isomer) 59.5:40.1]) : starting material 99.1 : 0.9. Then the solvent was removed at 38°C. A brownish solid was obtained. The solid was dissolved in 6.0 g ethyl acetate. The solution was added drop wise to a mixture of 11 g of aqueous sodium hydrogen carbonate (5 weight% solution in water), 11.0g of water, 17.4 g of ethyl acetate, 3.6 g of cyclohexane and 7.0 g of acetonitrile at 32°C. The obtained reaction mixture was stirred over night at ambient temperature, and then for 3 hours at 0°C. The precipitate of the blocked (protected) nucleoside was filtered off, washed with 6.0 g of water, and finally with 7.0 g of a mixture of acetonitrile and ethyl acetate (1:1).
Yield: 3.63 g; 40.1%.

### Example 3

A mixture of 5-fluoro-cytosine (1.0 g, 9.0 mmol), ammonium sulfate (0.12 g, 0.91 mmol), and hexamethyldisilazane (8.1 g, 50 mmol) was heated to reflux for 45 minutes until a clear solution was obtained. Some gas evolved (ammonia). The reaction mixture was cooled to 50°C, and concentrated in the vacuum to about half of the original volume. A turbid suspension was obtained. 13.2 g of dichloromethane, lithium trifluoromethane sulfonate (1.3 g, 8.3 mmol) and the "chloro sugar" C-137 (3.48 g, 8.1 mmol) were added. The beige mixture was stirred for 3 hours at ambient temperature (20-25°C), while it became brownish, products (anomeric mixture [(β-isomer) : (α-isomer) 55.6 : 44.4]) : starting material 99.0 : 1.0. Then the solvent was removed at 38°C. A brownish sticky solid was obtained. The solid was dissolved in 3.0 g ethyl acetate. The solution was added drop wise to a mixture of 11 g of aqueous hydrogen carbonate (5 weight% solution in water), 11.0 g of water, 8.7 g of ethyl acetate, 1.8 g of cyclohexane and 3.5 g of acetonitrile at 32°C. A three phase system was obtained. The reaction mixture was stirred over night at ambient temperature, and then filtered off, washed with 3.0 g of water, and finally with 3.6 g of a mixture of acetonitrile and ethyl acetate (1:1).
Yield: 0.78 g, 19.7%.

## Claims

1. Method of producing a free nucleoside compound by reacting a glycoside donor preferably a 1-halogen derivative, or 1-O-acyl, 1-0-alkyl, or an imidate preferably a trichloromethyl derivative, or a thio-alkyl derivative preferably a thiomethyl derivative of a blocked monosaccharide or oligosaccharide preferably ribose and 2-desoxyribose derivatives, said monosaccharide or oligosaccharide being blocked by removable protecting groups, with a protected nucleoside base, in a suitable anhydrous solvent and in the presence of a catalyst, whereby a blocked nucleoside compound is obtained, and removing the protecting groups from said blocked nucleoside compound in order to obtain the free nucleoside compound, **characterized in that** said catalyst is selected from the group comprising salts of an aliphatic sulphonic acid and/or salts of a optionally substituted aromatic sulphonic acid.

2. Method of producing a free nucleoside compound by reacting a glycoside donor preferably a 1-halogen derivative, or 1-O-acyl, 1-0-alkyl, or an imidate preferably a trichloromethyl derivative, or a thio-alkyl derivative of a blocked monosaccharide or oligosaccharide preferably ribose and 2-desoxyribose derivatives (said monosaccharide or oligosaccharide being blocked by removable protecting groups) with a protected nucleoside base, in a suitable anhydrous solvent and in the presence of a catalyst, whereby a blocked nucleoside compound is obtained, **characterized in that** said catalyst is selected from the group comprising salts of an aliphatic sulphonic acid and/or salts of a optionally substituted aromatic sulphonic acid.

3. Method according to claim 1 or 2, **characterized in that** the catalyst is chosen from a salt of an aliphatic sulphonic acid, preferably of methylsulphonic acid or of ethylsulphonic acid, or of a fluorinated aliphatic sulfonic acid, and preferably is a salt of trifluoromethane-sulfonic acid, pentafluoroethyl-sulfonic acid, or heptafluoropropyl-sulfonic acid, preferably a salt of methylsulphonic acid or a salt of trifluoromethanesulfonic acid.

4. Method according to claim 3, **characterized in that** the catalyst is an alkali salt or an earth alkali salt, preferably the lithium salt, preferably lithium methylsulphonic acid (lithium mesylate) or lithium-trifluoromethanesulfonate (LiOTf, lithiumtriflate).

5. Method according to claim 1 or 2, **characterized in that** the catalyst is a salt of scandium, preferably Sc(OTf)₃ or of copper preferably Cu(OTf)₂ or of magnesium preferably Mg(OTf)₂.

6. Method according to claim 1 or 2, **characterized in that** the catalyst is a salt of a known, optionally substituted, aromatic sulphonic acid, preferably an alkali salt or an earth alkali salt thereof, preferably an alkali salt or an earth alkali salt of benzenesulphonic acid, toluenesulphonic acid, and known related acids, preferably the lithium salt of said aromatic sulphonic acids.

7. Method according to any one of the claims 1-6, **characterized in that** the solvent to carry out the reaction is selected from the group comprising chlorinated solvents, preferably dichloromethane, dichloroethane, chloroform, chlorobenzene, or toluene, xylcl, acetonitril and/or propylene carbonate or related solvents, and preferably is a chlorinated solvent.

8. Method according to any one of the claims 1-7, **characterized in that** the reaction is carried out in the presence of lithium-trifluoromethanesulfonate in a chlorinated solvent, preferably in dichloromethane, dichloroethane, chloroform and/or chlorobenzene.

9. Method according to any one of the claims 1-8, **characterized in that** the protecting groups of blocked monosaccharides or oligosaccharides are selected from (C₁-C₈)alkylcarbonyl, or optionally substituted phenylcarbonyl, or optionally substituted benzylcarbonyl, preferably from (C₁-C₄)alkylcarbonyl, or optionally substituted phenylcarbonyl, preferably phenylcarbonyl, tolylcarbonyl, xylylcarbonyl or benzylcarbonyl; and preferably is acetyl or p-chloro-phenylcarbonyl.

10. Method according to any one of the claims 1-9, **characterized in that** the substituents 1-O-acyl, 1-0-alkyl and 1-halogen attached to the blocked monosaccharide or oligosaccharide are preferably substituents of the formulae -O-acyl(C₁-C₈), -O-alkyl(C₁-C₈) or halogen, preferably chlorine.

11. Method according to claim 10, **characterized in that** the substituents 1-O-acyl, 1-O-alkyl and 1-halogen are -O-acyl(C₁-C₄), -O-alkyl(C₁-C₄) or chlorine, preferably -O-(O)CCH₃ or chlorine, preferably chlorine.

12. Method according to any one of the claims 1-11, **characterized in that** the blocked monosaccharides or oligosaccharides are derived from ribose, 2-deoxyribose, arabinose, and glucose and wherein preferably all free hydroxyl groups are blocked with known protecting groups, preferably with the blocking groups selected from (C₁-C₈)alkylcarbonyl, or optionally substituted phenylcarbonyl, or optionally substituted benzylcarbonyl.

13. Method according to any one of the claims 1-12, **characterized in that** the protected nucleoside base is protected by a removable protecting group known per se, and is preferably protected by a trimethylsilyl (TMS)-group.

14. Method according to any one of the claims 1-13, **characterized in that** the nucleoside base is a halogen derivative, preferably Fluor derivatives, or is a heterocyclic compound containing five or six atoms, said heterocyclic ring containing one, two or three nitrogen atoms.

15. Method according to any one of the claims 1-14, **characterized in that** the nucleoside base is a halogen derivative, preferably Fluor derivatives, or is derived from the group comprising the following heterocyclic compounds, wherein these compound optionally may be substituted: uracil, cytosine preferably 5-azacytosine, 6-azauracil, 2-thio-6-azauracil, thymine, N-acyl-adenine, guanine, lumazine, imidazole, pyrazine, thiazole and triazole.

16. Method according to any one of the claims 1-15, **characterized in that** in the nucleoside obtained, the sugar residue is linked to the nitrogen atom to form a beta-glycoside.
